# EUROPEAN PATENT APPLICATION

(11) **EP 2 263 672 A1**
(43) Date of publication of application: **22.12.2010**
(21) Application number: 09305456.7
(22) Date of filing: 19.05.2009
(51) Int. Cl.: A61K 31/4439, C07D 401/06, A61P 25/00

(54) **Novel pharmaceutically acceptable salts of 4-(1H-imidazol-4-ylmethyl)pyridine and their therapeutical uses**

(71) Applicant: BIOPROJET, 75003 Paris (FR)
(72) Inventor: Capet, Marc, 35520 Melesse (FR); Labeeuw, Olivier, 35300 Fougeres (FR); Berrebi-Bertrand, Isabelle, 35740 Pace (FR); Robert, Philippe, 35740 Pace (FR); Ligneau, Xavier, 35760 Saint Gregoire (FR); Lecomte, Jeanne-Marie, 75003 Paris (FR); Schwartz, Jean-Charles, 75014 Paris (FR)
(74) Representative: Domenego, Bertrand

(57) **Abstract**

The present invention concerns novel pharmaceutical compositions of immethridine, in particular of novel pharmaceutically acceptable salts thereof, such as the dioxalate salt of immethridine, as well as its therapeutical uses and novel process of preparation.

## Description

Activators of the histamine H3 receptor (H3R) tend to inhibit the release of endogenous histamine from its stores in brain or the gastrointestinal tract. They represent potentially important drugs for the treatment of a variety of disorders in the CNS such as sleep disorders or neuropsychiatric disorders as well as in peripheral tissues such as gastrointestinal or cardiovascular systems (Celanire S. et al. in "The Third Histamine Receptor" D. Vohora, ed., CRC Press, 2009, pp. 103-165).

However, H3R activators, i.e. histamine full or partial agonists, known so far are imidazole derivatives which display a number of drawbacks attributable to the presence of the imidazole nucleus.

First, they are, like histamine itself, good substrates for histamine N-methyltransferase (HMT), a metabolizing enzyme abundant in human liver, which ensures an efficient first-pass inactivation of these compounds via transfer of a methyl group in *tele* position. N-methyltransferase enzyme is a major metabolizing enzyme in the brain of animal species including man. Both localizations tend to prevent or, at least, shorten the actions of imidazole-containing H3R activators.

Second, imidazole-containing drugs are often potent inhibitors and/or inducers of cytochrome P450 enzymes, a drawback for therapeutic applications since P450 cytochrome inhibition or induction results in potential drug-drug interactions which complicate co-treatments. Therefore, during the last decades, the efforts of medicinal chemists in the H3R ligand field have been directed towards the discovery of non-imidazole containing compounds (Celanire et al. *ibid*.).

Third, some H3R activators may present physicochemical properties incompatible or hardly compatible with their use as drugs, e.g. chemical instability or hygroscopic character.

The above drawbacks may explain why none of imidazole containing H3R activators has reached the level of therapeutic applications in humans.

The 4-(1*H*-imidazol-4-ylmethyl)pyridine, also known as immethridine, was disclosed by van der Stoel et al., Red. Trav. Chem., Pays-Bas, 102, 364-367, 1983. Its H3R activity was reported by Celanire S. et al. (XXXVth Annual Meeting of the European Histamine Research Society, Delphi, Greece, 2006, Poster P5), Wijtmans et al. (15th Noordwijkerhout-Camerino Symposium, Noordwijkerhout, the Netherlands, 2005, Poster); the corresponding dihydrobromide was taught by Kitbunnadaj R. et al. (J. Med. Chem. 2004, 47, 2414-2417).

A series of related compounds with alpha 2 adrenergic activities were also further described in a patent application (US 6,465,486) although salts of immethridine and their H3R activity are neither described nor suggested therein.

Although the *in vitro* H3 activity of 4-(1*H*-imidazol-4-ylmethyl)pyridine was reported by Celanire et al. and Wijtmans et al. (see supra), these authors merely considered this compound as a tool and not as a possible drug candidate. This is presumably due to the expected drawbacks of such imidazole containing skeleton, in accordance with the common general knowledge as discussed above. More particularly, the drug was shown by Celanire et al to be of low *in vivo* activity, even when administered intraperitoneally to rats, a species with a low hepatic HMT activity: doses as high as 10 mg/kg were found necessary to modestly decrease histamine in brain; this suggested that, in humans, the orally-administered drug should be even less potent, due to extensive hepatic first pass inactivation in a species with very high HMT activity. In any case, interactions of immethridine with HMT and its lack of significant toxicity were not reported, thereby failing to uncover the potential of the drug for clinical applications in human therapeutics.

Altogether, the teachings of the prior art confirm that immethridine could not possibly be considered as a drug candidate, but could merely be used as a research tool.

The present inventors have now surprisingly found that, against all expectations, immethridine or its pharmaceutically acceptable salts were suitable for human therapeutics. In particular, they have found that salts of immethridine show unexpected properties which make them extremely potent H3R activators *in vivo* and appear as drugs usable in human therapeutics, being surprisingly devoid of the above drawbacks.

More precisely, it has now been discovered that the oxalate salts of 4-(1*H-*imidazol-4-ylmethyl)pyridine, in particular the dioxalate salt, exhibit unexpected properties over immethridine or other salts of immethridine, such as stability, decreased hygroscopy and purification capabilities, and display all properties required to be considered as extremely potent drugs useful in several areas of human therapeutics.

According to one of its objects, the present invention thus concerns the pharmaceutical compositions comprising immethridine or one of its pharmaceutically acceptable salts, preferably comprising a pharmaceutically acceptable salt of immethridine and more preferably the oxalate salts of immethridine, in particular the dioxalate salt of immethridine of formula (I):

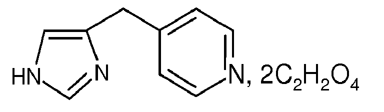

According to a further object, the present invention also concerns the oxalate salts of immethridine, such as the monooxalate or dioxalate salt of immethridine, preferably the dioxalate salt of immethridine.

Immethridine dioxalate is a white crystalline powder of MW= 339.26 g. It is herein referred to as BP1.5375.

According to a further object, the present invention also concerns the process of preparation of oxalate salts of immethridine, such as dioxalate. Said process comprises the step of reacting immethridine with oxalic acid. Preferably, the reaction is carried out in a suitable organic solvent such as propan-2-ol or other suitable alcohol, acetone or other suitable ketone. Generally, the ratio of the concentration of immethridine and oxalic acid depends on the salification degree that is desired. In particular, if the monooxalate salt is desired, equimolar quantities of immethridine and oxalic acid are used. If the dioxalate salt is desired twice more oxalic acid than immethridine is used.

Immethridine may be prepared by applying or adapting any known methods, such as those disclosed in US 6,465,486, Kitbunnadaj et al., J. Med. Chem. 2004, 47, 2414-2417, J. Med. Chem. 2003, 46, 5445-5457, Wijtmans et al. 15th Noordwijkerhout-Camerino Symposium, Noordwijkerhout, the Netherlands, 2005, Poster, Vaccaro et al., BMCL 2006, 16, 395-399, Alcalde et al., Tetrahedron 52, 48, 15197-15208, 1996.

According to a preferred aspect, the process of the invention comprises the step of preparing immethridine by coupling a compound of formula (II) : 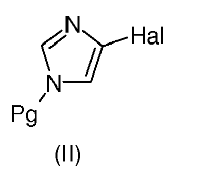 with a compound of formula (III) : 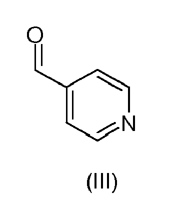 where Hal represents a halogen atom, such as iodide, Pg represents a protecting group of amino, such as trityl (triphenylmethyl), dimethylsulfamoyle, etc., so as to obtain the compound of formula : 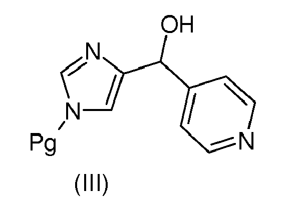 followed by deoxygenating/deprotecting said protecting group from the obtained compound (III).

Said coupling is generally conducted according to a metal exchange reaction, in the presence of EtMgBr, according to standard procedures. Generally, reagents are added at temperatures between -20°C and 0°C, and the reactional mixture may then be allowed to warm at room temperature.

Said deoxygenation/deprotection reaction is advantageously conducted by any suitable deoxygenation/deprotection means such as HI (iodhydric acid), hydrogenation using transition metal such as Pd(OH)₂ as catalyst, triethylsilane/trifluoroacetic acid, borane/dimethylsulfide or NaBH₄/trifluoroacetic acid, preferably HI.

The reaction may generally be carried out at a temperature comprised between room temperature and reflux temperature of the reaction mixture.

Said deoxygenation/deprotection reaction may advantageously be conducted after isolating the product obtained by the coupling reaction.

The following scheme illustrates a preferred embodiment of the process of the invention.

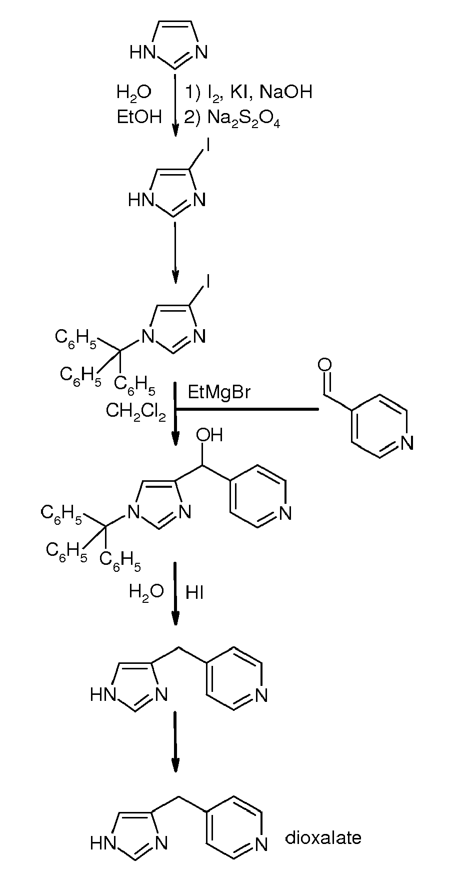

Said process of the invention also comprises any isolation and/or purification step of the desired compound of formula (I).

The starting products and/or reagents may be commercially available, or may be readily prepared by the skilled person by applying or adapting the procedures disclosed in the experimental part below.

Another interesting property related to industrial synthesis of the drug is the easier purification during crystallisation of the oxalate salts compared with the dihydrobromide. The present inventors have surprisingly discovered that imidazole, the main contaminant stemming from the synthesis of immethridine, is better removed by forming the dioxalate, than by forming the dihydrobromide as apparent from the experimental part below.

The present invention also provides pharmaceutical compositions comprising a pharmaceutically acceptable carrier and a therapeutically effective amount of an oxalate salt of immethridine.

As used herein, the term "*patient*" refers to a warm-blooded animal such as a mammal, preferably a human or a human child, which is afflicted with, or has the potential to be afflicted with one or more diseases and conditions described herein.

As used herein, the expression "*therapeutically effective amount*" refers to an amount of a compound of the present invention which is effective in reducing, eliminating, treating or controlling the symptoms of the herein-described diseases and conditions. The term "*controlling*" is intended to refer to all processes wherein there may be a slowing, interrupting, arresting, or stopping of the progression of the diseases and conditions described herein, but does not necessarily indicate a total elimination of all disease and condition symptoms, and is intended to include prophylactic treatment and chronic use.

As used herein, the expression "*pharmaceutically acceptable*" refers to those compounds, materials, compositions, or dosage forms which are, within the scope of sound medical judgment, suitable for contact with the tissues of human beings and animals without excessive toxicity, irritation, allergic response, or other problem complications commensurate with a reasonable benefit/risk ratio.

As used herein, the expression "*pharmaceutically acceptable salts*" refers to derivatives of the disclosed compounds wherein the parent compound is modified by making acid or base salts thereof. The pharmaceutically acceptable salts include the conventional non-toxic salts or the quaternary ammonium salts of the parent compound formed, for example, from non-toxic inorganic or organic acids. For example, such conventional non-toxic salts include those derived from inorganic acids such as hydrochloric, hydrobromic, sulfuric, sulfamic, phosphoric, nitric and the like; and the salts prepared from organic acids such as acetic, propionic, succinic, tartaric, citric, methanesulfonic, benzenesulfonic, glucoronic, glutamic, benzoic, salicylic, toluenesulfonic, oxalic, fumaric, maleic, and the like. Further addition salts include ammonium salts such as tromethamine, meglumine, epolamine, etc., metal salts such as sodium, potassium, calcium, zinc or magnesium.

The pharmaceutically acceptable salts of the present invention can be synthesized from the parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two. Generally, non-aqueous media like ether, ethyl acetate, ethanol, isopropanol, or acetonitrile are preferred. Lists of suitable salts are found in Remington's Pharmaceutical Sciences, 17th ed., Mack Publishing Company, Easton, PA, 1985, p. 1418 and P.H. Stahl, C.G. Wermuth, Handbook of Pharmaceutical salts - Properties, Selection and Use, Wiley-VCH, 2002, the disclosures of which are hereby incorporated by reference.

According to a still further object, the present invention is also concerned with an oxalate salt of immethridine as defined above for activating the H3R receptors, in particular for treating and/or preventing H3R related disorders, including stress, pain, psychosomatic disorders, insomnia, migraines, respiratory, allergic or inflammatory conditions (asthma, bronchitis, rhinitis, tracheitis, and the like), cardiac conditions (myocardial dysfunction and infarction), gastrointestinal conditions as a result of their antisecretory and anti-inflammatory actions (gastric and duodenal ulcers, gastro-oesophageal reflux, ulcerative colitis, Crohn's disease, irritable bowel, faecal incontinence, and the like), conditions of the urogenital system (cystitis, metritis, premenstrual syndrome, prostatic inflammations, urinary incontinence, genital disorders) and conditions of the cutaneous system (urticaria, itching), arthritis and other rheumatic conditions, conjunctivitis and other ocular inflammations, sialorrhoea, secretion, inflammation, sleep and circadian rhythm disorders, convulsions, hypothalamohypophyseal secretion disorders, depressive states, cerebral circulation disorders, immune system disorders, allergic conditions.

In particular, the compounds of the invention may be used as a psychotropic, hypnotic, sleep regulator agent and/or anaesthesia adjuvant.

Unexpectedly, the present inventors have also discovered that the oxalate salts of immethridine surprisingly prevent the adverse events associated with chronic use of GABA-A receptor positive modulators, such as sleep pattern disorders including sleep onset latency, decrease in deep sleep, disorders of circadian rhythms, relative insomnia such adverse events being mainly triggered by abrupt cessation of such chronic use.

Such GABA-A receptor positive modulators include hypnotic drugs such as benzodiazepines, imidazopyridines, such as Zolpidem, barbiturates, sedative drugs, alcohol.

The present invention thus provides for an oxalate salt of immethridine for the prevention and/or treatment of abstinence symptoms, withdrawal syndrome, and dependence from drugs, benzodiazepine receptor agonists, and/or alcohol..

According to a still further object, the present invention also provides for combinations comprising an oxalate salt of immethridine with a GABA-A receptor positive modulator.

According to a still further object, the present invention also concerns the methods of treatment comprising administering an effective amount of an oxalate salt of immethridine for treating and/or preventing the above conditions or disorders.

The identification of those subjects who are in need of treatment of herein-described diseases and conditions is well within the ability and knowledge of one skilled in the art. A clinician skilled in the art can readily identify, by the use of clinical tests, physical examination and medical/family history, those subjects who are in need of such treatment.

A therapeutically effective amount can be readily determined by the attending diagnostician, as one skilled in the art, by the use of conventional techniques and by observing results obtained under analogous circumstances. In determining the therapeutically effective amount, a number of factors are considered by the attending diagnostician, including, but not limited to: the species of subject; its size, age, and general health; the specific disease involved; the degree of involvement or the severity of the disease; the response of the individual subject; the particular compound administered; the mode of administration; the bioavailability characteristic of the preparation administered; the dose regimen selected; the use of concomitant medication; and other relevant circumstances.

The amount of a compound of the invention, which is required to achieve the desired biological effect, will vary depending upon a number of factors, including the dosage of the drug to be administered, the chemical characteristics (e.g. hydrophobicity) of the compounds employed, the potency of the compounds, the type of disease, the diseased state of the patient, and the route of administration.

In general terms, the compounds of this invention may be provided in an aqueous physiological buffer solution containing 0.1 to 10% w/v compound for parenteral administration. Typical dose ranges are from 0.1 µg/kg to 0.1 g/kg of body weight per day; a preferred dose range is from 0.001 mg/kg to 1 mg/kg of body weight per day, preferably from 0.001 mg/kg to 0.1 mg/kg of body weight per day. A preferred daily dose for adult humans includes 1, 5, 50, 100 and 200 µg, and an equivalent dose in a human child. The preferred dosage of drug to be administered is likely to depend on such variables as the type and extent of progression of the disease or disorder, the overall health status of the particular patient, the relative biological efficacy of the compound selected, and formulation of the compound excipient, and its route of administration.

The compounds of the present invention are capable of being administered in unit dose forms, wherein the terms "*unit dose*" mean a single dose which is capable of being administered to a patient, and which can be readily handled and packaged, remaining as a physically and chemically stable unit dose comprising either the active compound itself, or as a pharmaceutically acceptable composition, as described hereinafter. As such, typical preferred daily dose ranges are from 0.001 to 0.1 mg/kg of body weight. By way of general guidance, unit doses for humans range from 0.01 mg to 10 mg per day. Preferably, the unit dose range is from 0.005 to 5 mg administered one to four times a day, and even more preferably from 0.01 mg to 1 mg, once a day. Compounds provided herein can be formulated into pharmaceutical compositions by admixture with one or more pharmaceutically acceptable excipients. Such compositions may be prepared for use in oral administration, particularly in the form of tablets, coated tablets, orodispersible preparations such as Lyoc formulations, or capsules; or parenteral administration, particularly in the form of liquid solutions, suspensions or emulsions; or intranasally, particularly in the form of powders, nasal drops, or aerosols; or dermally, for example, topically or via trans-dermal patches or ocular administration, or intravaginal or intra-uterine administration, particularly in the form of pessaries or by rectal administration.

The compositions may conveniently be administered in unit dosage form and may be prepared by any of the methods well known in the pharmaceutical art, for example, as described in Remington: The Science and Practice of Pharmacy, 20th ed.; Gennaro, A. R., Ed.; Lippincott Williams & Wilkins: Philadelphia, PA, 2000. Pharmaceutically compatible binding agents and/or adjuvant materials can be included as part of the composition. Oral compositions will generally include an inert diluent carrier or an edible carrier.

The tablets, pills, powders, capsules, orodispersible preparations, troches and the like can contain one or more of any of the following ingredients, or compounds of a similar nature: a binder such as microcrystalline cellulose, or gum tragacanth; a diluent such as starch or lactose; a disintegrant such as starch and cellulose derivatives; a lubricant such as magnesium stearate; a glidant such as colloidal silicon dioxide; a sweetening agent such as sucrose or saccharin; or a flavoring agent such as peppermint, or methyl salicylate. Capsules can be in the form of a hard capsule or soft capsule, which are generally made from gelatin blends optionally blended with plasticizers, as well as a starch capsule. In addition, dosage unit forms can contain various other materials that modify the physical form of the dosage unit, for example, coatings of sugar, shellac, or enteric agents. Other oral dosage forms syrup or elixir may contain sweetening agents, preservatives, dyes, colorings, and flavorings. In addition, the active compounds may be incorporated into fast dissolve, modified-release or sustained-release preparations and formulations, and wherein such sustained-release formulations are preferably bi-modal.

Preferred formulations include pharmaceutical compositions in which a compound of the present invention is formulated for oral or parenteral administration, or more preferably those in which a compound of the present invention is formulated as a tablet. Preferred tablets contain lactose, cornstarch, magnesium silicate, croscaramellose sodium, povidone, magnesium stearate, or talc in any combination. It is also an aspect of the present disclosure that a compound of the present invention may be incorporated into a food product or a liquid.

Liquid preparations for administration include sterile aqueous or non-aqueous solutions, suspensions, and emulsions. The liquid compositions may also include binders, buffers, preservatives, chelating agents, sweetening, flavoring and coloring agents, and the like. Non-aqueous solvents include alcohols, propylene glycol, polyethylene glycol, acrylate copolymers, vegetable oils such as olive oil, and organic esters such as ethyl oleate. Aqueous carriers include mixtures of alcohols and water, hydrogels, buffered media, and saline. In particular, biocompatible, biodegradable lactide polymer, lactide/glycolide copolymer, or polyoxyethylene-polyoxypropylene copolymers may be useful excipients to control the release of the active compounds. Intravenous vehicles can include fluid and nutrient replenishers, electrolyte replenishers, such as those based on Ringer's dextrose, and the like. Other potentially useful parenteral delivery systems for these active compounds include ethylene-vinyl acetate copolymer particles, osmotic pumps, implantable infusion systems, and liposomes.

Alternative modes of administration include formulations for inhalation, which include such means as dry powder, aerosol, or drops. They may be aqueous solutions containing, for example, polyoxyethylene-9-lauryl ether, glycocholate and deoxycholate, or oily solutions for administration in the form of nasal drops, or as a gel to be applied intranasally. Formulations for buccal administration include, for example, lozenges, Lyoc formulations or pastilles and may also include a flavored base, such as sucrose or acacia, and other excipients such as glycocholate. Formulations suitable for rectal administration are preferably presented as unit-dose suppositories, with a solid based carrier, such as cocoa butter, and may include a salicylate. Formulations for topical application to the skin preferably take the form of an ointment, cream, lotion, paste, gel, spray, aerosol, or oil. Carriers which can be used include petroleum jelly, lanolin, polyethylene glycols, alcohols, or their combinations. Formulations suitable for transdermal administration can be presented as discrete patches and can be lipophilic emulsions or buffered, aqueous solutions, dissolved and/or dispersed in a polymer or an adhesive.

Alternative administrations include also solutions, ointments or other formulations acceptable for ocular administration.

According to a particular aspect, the compounds of the invention may be administered by the cutaneous, ocular or inhalation route as disclosed above.

Other features of the invention will become apparent in the course of the following description of exemplary embodiments that are given for illustration of the invention and not intended to be limiting thereof.

### EXAMPLES

### Example 1 : Preparation of BP1.5375

### Pyridin-4-yl-(1-trityl-1H-imidazol-4-yl)-methanol

A three-neck round-bottom flask fitted with an a thermometer was charged with 4-iodo-1-triphenylmethyl-1H-imidazole (5 g, 11.5 mmol) and anhydrous dichloromethane (30 mL), under argon and cooled to -10°C. After addition of ethyl magnesium bromide (3.82 mL, 11.5 mmol, 3M in Et₂O), the mixture was stirred for 90 minutes at -10°C. Then 4-pyridinecarboxaldehyde (0.972 mL, 10.3 mmol, 0.9 eq) was added dropwise via syringe at -10°C. After stirring for 30 minutes at -10°C, the solution was allowed to warm at room temperature and stirred for 30 minutes again. After quenching with a saturated solution of NH₄Cl and water, the mixture was extracted twice with dichloromethane. The combined organic layers were dried (MgSO₄) and concentrated under reduced pressure. The residue was triturated with iPr₂O, filtered and oven-dried to afford pyridin-4-yl-(1-trityl-1H-imidazol-4-yl)-methanol as an off-white solid used without further purification

### 4-(1H-imidazol-4-yl-methyl)-pyridine

A round-bottom flask equipped with a refluxer was charged with crude pyridin-4-yl-(1-trityl-1 H-imidazol-4-yl)-methanol and 57 wt% aq HI (75 mL). The mixture was refluxed overnight, cooled to room temperature and poured on crushed ice and a few grams of NaHSO₃. Extraction of the mixture with dichloromethane (twice) removed the formed triphenylmethane. The aqueous solution was basified with solid K₂CO₃ and extracted several times with dichloromethane and ethyl acetate. The combined organic layers were dried (MgSO₄) and concentrated under reduced pressure to afford crude 4-(1H-imidazol-4-yl-methyl)-pyridine as a pale yellow solid.

### BP1.5375:4-(1H-imidazol-4-ylmethyl)-pyridine, dioxalate

To a solution of 4-(1H-imidazol-4-yl-methyl)-pyridine (159 mg, 1 mmol) in propan-2-ol (4 mL) was added a solution of oxalic acid (180 mg, 2 mmol, 2 eq) in the same solvent (1 mL). After stirring for 1 h at room temperature, the resulting salt was filtered and oven-dried to afford pure 4-(1H-imidazol-4-ylmethyl)-pyridine dioxalate.

¹H NMR (DMSO-d⁶, 250MHz): 13.59 (br s, 5H), 8.79 (s,1H), 8.48 (d, 6.0Hz, 2H ), 7.39 (s, 1 H), 7.28 (d, 6.0Hz, 2H ), 4.06 (s, 2H).

¹³C NMR (DMSO-d⁶, 63MHz): 164.0, 150.2, 148.4, 135.4, 131.9, 124.9, 117.9, 30.2

Anal. Calcd for C₁₃H₁₃N₃O₈: C, 46.02%; H, 3.86%; N, 12,39%. Found: C, 46.05%; H, 3.85%; N, 12,63%.

### Example 2 : Properties of the immethridine dioxalate

A batch of crude immethridine base containing 4% of imidazole leads, after single crystallisation of the immethridine dioxalate in aqueous isopropanol, to a salt containing only 0.7% of imidazole salt. In contrast, the immethridine dihydrobromide does not crystallise in aqueous isopropanol. In dry isopropanol, the obtained immethridine dihydrobromide, which is treated with diisopropyl oxyde, is contamined with 1.35% of imidazole. Furthermore, making a hydrobromide in isopropanol can lead to the formation of isopropylbromide, an alkylating agent that is potentially cancerogenic.

Immethridine dioxalate is a stable compound: when heated at 80°C during one week, no apparent degradation was detected by HPLC analysis. On the opposite, immethridine darkens, showing a lack of stability. In addition, in contrast with immethridine and its dihydrobromide, which are hygroscopic (they take up to 50-130% of their weight and often become deliquescent when left for a few days in a water-saturated atmosphere at room temperature), immethridine dioxalate does not take up more than 13-15% water under similarly harsh conditions, indicating a significant superiority in terms of pharmaceutical manufacturing operation opportunities.

### Example 3 : Biological activity

### 1. In vitro data

■ BP1.5375 is a potent activator of the human H3R: on the recombinant hH3R expressed in HEK 293 cells, it activates [³⁵S]GTPγS binding with an EC50 of ∼1 nM and an intrinsic activity of 0.75 compared with histamine. In contrast, it is relatively less potent as an activator of β arrestin translocation, with an EC50 of ∼17 nM and an intrinsic activity of 0.5, compared to histamine, suggesting its reduced ability to desensitize the same receptor.
■ BP1.5375 does not interact with the hERG potassium channel. Indeed, BP1.5375 at concentrations up to 10 µM is found not to inhibit the [³H]dofetidide binding assay and the potassium hERG current in the functional patch clamp assay using, in the two tests, HEK-293 cells stably expressing the hERG potassium channel. This property is essential to ensure lack of cardiotoxicity related to QT prolongation of the electrocardiogram.
■ Unexpectedly for an H3R agonist, BP1.5375 is a poor substrate of human HMT. This was established using the cytosol of fresh human hepatocytes as an enzyme source, 20 µM (S)-adenosyl methionine as the methyl donor and BP1.5375 (or histamine or (R)α-methyl histamine) in increasing concentrations as substrate(s); the corresponding *tele* methyl derivative(s) formed from 0 to 90 min incubations were separated and quantified by HPLC-MS and Michaelis constants and maximal velocity calculated by Lineweaver-Burk analysis of the data. Table 1 shows such data which allow to calculate the specificity constant, i.e. the ratio of Vm/Km which were 0.048, 0.034 and 0.0013 for histamine, (R)α-methyl histamine and BP1.5375, respectively at the physiological pH of 7.4.

| | pH 7.4 | |
|---|---|---|
| | Km (µM) | Vm (µM/min) |
| Histamine | 3.93 | 0.19 |
| (R)α-methyl histamine | 16.26 | 0.56 |
| BP1.5375 | 3.31 | 0.004 |

This indicates that BP1.5375 is methylated at an extremely low rate, about 50 to 150-fold lower than histamine or the prototypical H3R agonist (R)α-methyl histamine, respectively.

### 2. In vivo data

i) The reduced *in vivo* inactivation of BP1.5375 by transmethylation in mouse brain, a tissue rich in HMT, was shown by measuring the AUCs of the unchanged and *tele* methylated derivative after administration of 1 mg/kg, p.o. : the inactive metabolite represented only 23% of the parent drug. (R)α-methyl histamine by itself poorly penetrates into the brain (Yamazaki et al., J. Pharm. Pharmacol., 1994, 46, 371-74), however the corresponding percentage for *tele* methylated (R)α-methyl histamine in mouse blood was 150 % (Rouleau et al., JPET, 1997, 281, 1085-94).
   The same experiment showed that BP1.5375 crosses rather well the blood-brain barrier with a ratio of its brain/blood AUCs close to unit. In Sprague-Dawley, rats receiving 1 mg/kg p.o. of the drug data led to similar conclusions: the ratio of AUCs of metabolite/unchanged drug in brain was only 19.6% and the brain/blood ratio of 2.6.
   BP1.5375 given orally to rats or mice was shown to be an extraordinarily potent activator of the H3R in brain: it reduced the activity of histaminergic neurons, assessed by the reduction of levels of *tele* methylhistamine, a major extracellular metabolite, with ED50 values as low as ∼30 micrograms/kg. This response is maintained upon repeated administration over 7 days, indicating that chronic treatment indications in humans may be envisaged.
   At the same very low dosages, the drug enhanced significantly the duration of deep SWS (slow wave sleep) period when administered 30 min before the start of the light-on period to rats chronically equipped with an EEG recording device (24-hr recordings analyzed according to Louis et al., J. Neurosci. Methods, 2004, 133, 71-80). In the marmoset, a species with a sleep pattern more similar to that of humans, oral doses as low as 0.01 mg/kg were sufficient to enhance the proportion of deep sleep over light sleep, thereby confirming the sleep-promoting activity of the compound.
   While the sleep-promoting activity of several other H3R agonists has been previously described (Lin et al., Brain Res., 1990, 523, 325-330; Monti et al., Neuropsychopharmacology, 1996, 15, 31-35), much higher dosages - by two to three orders of magnitude - were required to obtain this effect. In addition, it has been discovered that the sleep-promoting effect of BP1.5375 does not diminish upon repeated o.d. administration over several days; this lack of tolerance contrasts with the rapid development of tolerance (over only a few days in the same rats) to the hypnotic effect of Zolpidem, a currently used benzodiazepine site ligand.
   Furthermore, it has been discovered that BP1.5375 does not give rise to a rebound phenomenon upon interruption of a repeated treatment. Accordingly, after interruption of a 6-day treatment of rats with 0.3 mg/kg, p.o. of this drug, it has been found that the sleep pattern is re-established as it was before treatment. This is in contrast with the long-lasting disorders of circadian rhythms, namely the relative insomnia, which occur at the interruption of a repeated treatment with benzodiazepines or imidazopyridines such as Zolpidem. Accordingly, after interruption of a 15-day treatment of rats with 20 mg/kg, p.o. Zolpidem withdrawal symptoms consist of increases in sleep onset latency, decrease in deep sleep.
   Such withdrawal symptomatology is known to also develop in humans and to be at the origin of the well-established pharmacodependence to benzodiazepines and other positive modulators of GABA-A receptor function such as imidazopyridines with hypnotic effects, barbiturates or alcohol.
   Even more, it has been discovered that administration of BP1.5375 when interrupting a repeated treatment with a positive modulator of GABA-A receptors prevents the occurrence of withdrawal symptoms that, otherwise, would appear under such circumstances. Accordingly, rats treated orally for 18 days by 20 mg/kg o.d. Zolpidem followed by 0.3 mg/kg, p.o., BP1.5375 failed to display on the next days the disturbed circadian rhythm characterized by delayed sleep onset and decreases in deep sleep.
ii) BP1.5375 also displays a good general tolerance since it does not induce any apparent toxicity sign in rodents at doses as high as 100 mg/kg, indicating a "safety margin" over three orders of magnitude.
iii) In contrast with many imidazole-containing derivatives, BP1.5375 does not interact significantly with the major classes of recombinant human cytochrome P450 isoforms, its IC50 values being 29.3, ≥100 and 3.1 micromolar at the 3A4, 2D6 and 2C9 isoforms, respectively. These values are over 3 orders of magnitude higher than the expected plasma or tissue concentrations at therapeutic dosage.
   In addition, the compound displays also low induction potential on human CYP 450 isoforms (3A4, 2C9, 2E1, 2B6) and UGTs, conjugating enzymes which are not induced in human hepatocytes at concentrations over 3 orders of magnitude higher than the expected therapeutic drug concentrations.
   These findings indicate that BP1.5375 represents an efficient treatment of the withdrawal syndrome that is associated with chronic administration of these various compounds: it will be profitably used in the prevention of the abstinence symptoms which develop at interruption of treatment of insomnia by various hypnotic drugs and will facilitate the treatment of alcohol abuse.

## Claims

1. A pharmaceutical composition comprising immethridine or one of its pharmaceutically acceptable salts.

2. A pharmaceutical composition according to claim 1, wherein immethridine is in the form of a pharmaceutically acceptable salt.

3. The pharmaceutical composition according to claim 1 or 2, wherein immethridine is in the form of an oxalate salt.

4. A pharmaceutical composition according to claim 1, 2 or 3, wherein immethridine is in the form of the dioxalate salt, of formula:

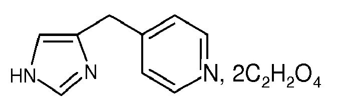

5. The oxalate salts of immethridine.

6. The compound according to claim 5, which is the dioxalate salts of immethridine, of formula (I):

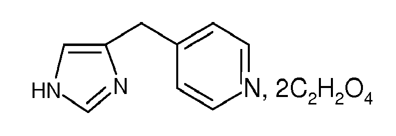

7. A process of preparation of the compound according to claim 5 or 6 comprising the step of reacting immethridine with oxalic acid in an organic solvent.

8. The process according to claim 7, which further comprises the steps of preparing immethridine by coupling a compound of formula (II) 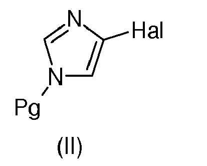 with a compound of formula (III) 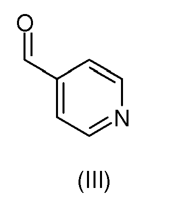 where Hal represents a halogen atom, Pg represents a protecting group followed by dexoxygenating/deprotecting said protecting group Pg.

9. Immethridine or one of its pharmaceutically acceptable salts for preventing and/or treating H3R related disorders.

10. Immethridine or one of its pharmaceutically acceptable salts according to claim 9, which is in the form of oxalate salts.

11. Immethridine or one of its pharmaceutically acceptable salts according to claim 9 or 10, which is the dioxalate salts of immethridine.

12. Immethridine or one of its pharmaceutically acceptable salts according to anyone of claims 9 to 11 for treating and/or preventing stress, pain, psychosomatic disorders, insomnia, migraines, respiratory, allergic or inflammatory conditions (asthma, bronchitis, rhinitis, tracheitis, and the like), cardiac conditions (myocardial dysfunction and infarction), gastrointestinal conditions as a result of their antisecretory and anti-inflammatory actions (gastric and duodenal ulcers, gastro-esophageal reflux, ulcerative colitis, Crohn's disease, irritable bowel, faecal incontinence, and the like), conditions of the urogenital system (cystitis, metritis, premenstrual syndrome, prostatic inflammations, urinary incontinence, genital disorders) and conditions of the cutaneous system (urticaria, itching), arthritis and other rheumatic conditions, conjunctivitis and other ocular inflammations, sialorrhoea, secretion, inflammation, sleep or circadian rhythm disorders, convulsions, hypothalamohypophyseal secretion disorders, depressive states, cerebral circulation disorders, immune system disorders, allergic conditions.

13. Immethridine or one of its pharmaceutically acceptable salts according to anyone of claims 9 to 11 as a psychotropic, hypnotic, sleep regulator agent and/or as an anaesthesia adjuvant.

14. Immethridine or one of its pharmaceutically acceptable salts according to anyone of claims 9 to 11 for the prevention or treatment of abstinence symptoms, withdrawal syndrome, and dependence from drugs, benzodiazepine receptor agonists, and/or alcohol.

15. Combinations comprising an oxalate salt of immethridine with a GABA-A positive modulator.

## Amended claims

### Amended claims in accordance with Rule 137(2) EPC.

1. A pharmaceutical composition comprising immethridine or one of its pharmaceutically acceptable salts, as well as their solvated, including hydrated forms, and polymorphic forms of said salts and/or polymorphic forms of said solvated (including hydrated) forms.

2. A pharmaceutical composition according to claim 1, wherein immethridine is in the form of a pharmaceutically acceptable salt.

3. The pharmaceutical composition according to claim 1 or 2, wherein immethridine is in the form of an oxalate salt.

4. A pharmaceutical composition according to claim 1, 2 or 3, wherein immethridine is in the form of the dioxalate salt, of formula:

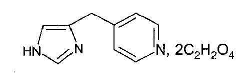

5. The salts of immethridine chosen from the dihydrochloride, dihydrogenosulfate, toluenesulfonate, ditoluenesulfonate, dicamphorsulfonate, dibenzenesulfonate, phosphate, dimethanesulfonate, alpha-ketoglutarate, oxalate, monosulfate salts of immethridine, as well as their solvated, including hydrated forms, and polymorphic forms of said salts and/or polymorphic forms of said solvated including hydrated forms.

6. The compound according to claim 5, which is the dioxalate salt of immethridine, of formula (I):

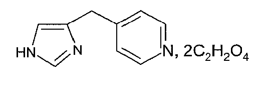

7. The compound according to claim 5 or 6 which is the dioxalate salt of immethridine in its polymorphic form III comprising a characterizing peak at 16.6° by X ray diffraction.

8. A process of preparation of the compound according to claim 5, 6 or 7 comprising the step of reacting immethridine with the corresponding acid in an organic solvent.

9. The process according to claim 8, which further comprises the steps of preparing immethridine by coupling a compound of formula (II) 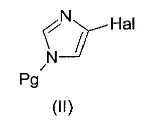 with a compound of formula (III) 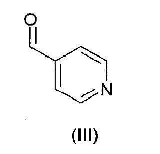 where Hal represents a halogen atom, Pg represents a protecting group followed by dexoxygenating/deprotecting said protecting group Pg.

10. Immethridine or one of its pharmaceutically acceptable salts as well as their solvated, including hydrated forms, and polymorphic forms of said salts and/or polymorphic forms of said solvated (including hydrated) forms for preventing and/or treating H3R related disorders.

11. Immethridine or one of its pharmaceutically acceptable salts according to claim 10, which is in the form of oxalate salts.

12. Immethridine or one of its pharmaceutically acceptable salts according to claim 10 or 11, which is the dioxalate salts of immethridine.

13. Immethridine or one of its pharmaceutically acceptable salts according to anyone of claims 10 to 12 for treating and/or preventing stress, pain, psychosomatic disorders, insomnia, migraines, respiratory, allergic or inflammatory conditions (asthma, bronchitis, rhinitis, tracheitis, and the like), cardiac conditions (myocardial dysfunction and infarction), gastrointestinal conditions as a result of their antisecretory and anti-inflammatory actions (gastric and duodenal ulcers, gastro-esophageal reflux, ulcerative colitis, Crohn's disease, irritable bowel, faecal incontinence, and the like), conditions of the urogenital system (cystitis, metritis, premenstrual syndrome, prostatic inflammations, urinary incontinence, genital disorders) and conditions of the cutaneous system (urticaria, itching), arthritis and other rheumatic conditions, conjunctivitis and other ocular inflammations, sialorrhoea, secretion, inflammation, sleep or circadian rhythm disorders, convulsions, hypothalamohypophyseal secretion disorders, depressive states, cerebral circulation disorders, immune system disorders, allergic conditions.

14. Immethridine or one of its pharmaceutically acceptable salts according to anyone of claims 10 to 12 as a psychotropic, hypnotic, sleep regulator agent and/or as an anaesthesia adjuvant.

15. Immethridine or one of its pharmaceutically acceptable salts according to anyone of claims 10 to 12 for the prevention or treatment of abstinence symptoms, withdrawal syndrome, and dependence from drugs, benzodiazepine receptor agonists, and/or alcohol.

16. Combinations comprising a pharmaceutically acceptable salt of immethridine as well as their solvated, including hydrated forms, and polymorphic forms of said salts and/or polymorphic forms of said solvated (including hydrated) forms with a GABA-A positive modulator.
